# EUROPEAN PATENT APPLICATION

(11) **EP 4 266 039 A1**
(43) Date of publication of application: **25.10.2023**
(21) Application number: 21851604.5
(22) Date of filing: 20.12.2021
(51) Int. Cl.: G01N 27/48, C12Q 1/30

(54) **METHOD FOR SELECTIVELY MEASURING A CONCENTRATION OF A COMPOUND IN A COMPLEX SAMPLE**

(30) Priority: 21.12.2020 ES 202031280
(71) Applicant: Bioquochem S.L., 33428 Llanera-Asturias (ES)
(72) Inventor: HEVIA SÁNCHEZ, David, 33428 Llaneras-Asturias (ES); MUÑOZ CIMADEVILLA, Henar, 33428 Llaneras-Asturias (ES); DÍAZ GONZÁLEZ, María, 33428 Llaneras-Asturias (ES)
(74) Representative: Tribalyte Ideas
(86) International application number: PCT/ES2021/070910
(87) International publication number: WO 2022/136712

(57) **Abstract**

The invention provides a method for measuring the concentration of a compound for analysis in an original sample. The method comprises placing a first compound (1, 6) in a first electrochemical transducer (2), measuring a first concentration by means of applying a potential in the electrochemical transducer (2), mixing the first compound with a second compound, placing the modified sample (5) in a second electrochemical transducer (2), and measuring a second concentration by means of applying a potential in the electrochemical transducer. The first or the second compound is a part of the original sample (1). Lastly, an operation is performed between the first and the second concentration to obtain the concentration of the compound for analysis in the original sample.

## Description

### FIELD OF THE INVENTION

The present invention is comprised among analytical methods for measuring molecules or enzyme activities in complex samples by means of the selective measurement of the concentration of hydrogen peroxide.

### BACKGROUND OF THE INVENTION

The measurement of the concentration of a given substance can sometimes be useful for evaluating the behavior of said substance in the event of certain actions.

One example is hydrogen peroxide, which is used in the chemical industry, food industry, etc. Because of its very widespread use, it is very useful to have methods which allow knowing its concentration in a sample.

There are also many other compounds for which the measurement of their concentration is advantageous.

Some methods intended for measuring concentrations of compounds in a sample are known.

Hydrogen peroxide (H₂O₂) is one of the most important substrates in numerous biological reactions catalyzed by multifunctional oxidases. H₂O₂ has several important properties, such as its oxidating property, energy source, decomposing gas formation, a free radical source, effects on biological processes, and its use in chemical synthesis. Depending on the exposure index (> 10%), H₂O₂ is an irritant for the skin, eyes, gastrointestinal tract, brain, and mucous membranes.

The precise determination of H₂O₂ is very important in the biological, pharmaceutical, clinical and environmental science, food processing, and textile fields. In industrial and medical areas, it is used for the treatment, bleaching, and sterilization of sewage. In addition to its role in cell cytotoxicity and redox status, H₂O₂ plays a key role as a secondary messenger, a vital and complex modulator of biological pathways, such as cell growth, cell differentiation, apoptosis, vascular remodeling, immune activation, stomatic movement, and root growth. High levels of H₂O₂ have been involved in disease including cancer, arthritis, diabetes, neurodegenerative disorders, cardiovascular diseases, asthma, age- and oxidative stress-related diseases.

The detection of H₂O₂ sets the groundwork for many analytical techniques and commercial test kits, such as colorimetry, fluorimetry, spectrophotometry, chemiluminescence, and electrochemical method. The main drawback is that the analyses take a long time, are expensive, and especially require prolonged prior treatments for preparing the samples, use of expensive reagents and interference.

In electrochemistry, H₂O₂ can be directly reduced / oxidized on solid surfaces of electrodes. The detection of H₂O₂ with conventional electrodes made of noble metals is primarily restricted by the interferences from other electroactive species coexisting in the real samples. In numerous research projects, the detection of H₂O₂ was primarily focused on modifications to the electrodes to solve the problem of low sensitivity and high overpotentials. To selectively manufacture an H₂O₂ biosensor, platinum nanoparticles, redox polymers, metal oxides, carbon nanofibers, carbon nanotubes, a wide range of advanced nanomaterials such as nanohybrids of carboxylic acid, fluorophores, graphene capsules, and reduced graphene oxide have been used. Electrodes made of tetraethyl orthosilicate, polydimethylsiloxane, and TTF-modified graphite disc have been used.

However, all the methods up until now have the same problem when dealing with complex samples, which are the interferences present when attempting to analyze H₂O₂ in complex samples such as biological fluids. The present invention intends to show a method which allows overcoming those interferences to obtain a measurement of H₂O₂ in a selective manner, and it intends for this measurement to allow other molecules and/or enzyme activities to be quantified.

### BRIEF DESCRIPTION OF THE INVENTION

The present invention provides an alternative solution to the problem proposed above by means of a method for measuring oxidation parameters according to claim 1. The dependent claims define preferred embodiments of the invention.

Unless defined otherwise, all the terms (both scientific and technical terms) used herein must be interpreted as one skilled in the art would interpret them. Therefore, it shall be understood that commonly used terms must be interpreted as one familiar with the art would interpret them, and not in an idealized or strictly formal manner.

Throughout the text, the word "comprises" (and its variants, such as "comprising") must not be understood in an exclusive manner, but rather must be understood in the sense that they allow the possibility that what is defined may include additional elements or steps.

One object of the present invention relates, although without limitation, to a method for measuring a concentration of a compound for analysis in an original sample, the method comprising the steps of
placing a first compound in a first electrochemical transducer containing a working electrode, a reference or pseudo-reference electrode, and an auxiliary electrode;
measuring a first concentration by means of applying a potential in the electrochemical transducer;
mixing the first compound with a second compound, where the first or the second compound is a part of the original sample, obtaining a modified sample, where the modified sample has a concentration of the compound for analysis different from that of the original sample, due to the action between the first compound and the second compound;
placing the modified sample in a second electrochemical transducer containing a working electrode, a reference or pseudo-reference electrode, and an auxiliary electrode;
measuring a second concentration by means of applying a potential in the electrochemical transducer;
performing an operation between the first and the second concentration to obtain the concentration of the compound for analysis in the original sample.

Therefore, by means of a simple operation it is possible to calculate the concentration of a compound for analysis within an original sample. The mixture between the first and the second compound, one of which is the sample to be analyzed, modifies the concentration of the compound for analysis in the sample. It is thereby possible to calculate, by the difference, those values intended to be calculated, without the presence of other compounds that are very common in biological fluids from interfering in said measurement.

The mixture of the first compound with the second compound can be performed using the same portion of first compound that intervened in the first measurement or a different portion, based on the variant of the method and on the results to be obtained.

Not all the information needed to calculate the concentration of the compound to be analyzed is obtained with a single measurement because the signal is affected by the reaction of the many compounds which may be contained in the sample. However, by performing selective measurements and operating with same according to the method of the invention, it is possible to obtain the desired measurement.

As a result of this method, it is possible to detect concentrations of hydrogen peroxide between 100 nM and 100 mM, which is a very broad range since conventional methods barely cover two orders of magnitude.

In particular embodiments, the step of measuring the first or the second concentration comprises
applying a voltage to the working electrode by means of a potentiostat according to an amperometry method, said voltage being comprised within the range between 0.35 and 0.45 V
obtaining a mean of several current intensity measurements obtained in a given time range.

Amperometry is a particular example which allows a rapid, simple, and cost-effective measurement in that voltage range. Nevertheless, another type of electrochemical techniques, such as square wave voltammetry, together with other voltage ranges, allow similar results to be obtained.

This particular example, in which the mean of several current intensity measurements is obtained, allows measurement variability to drop, attenuating any unusual measurement that may be obtained due to momentary disruptions to the system (the apparatus is hit, an electromagnetic interference from a nearby apparatus, shaking, etc.).

In particular embodiments, the time range is comprised in the range between 20 and 40 seconds, specifically between 25 and 30 seconds.

The method of the invention allows the relevant data to be calculated in less than 40 seconds. The range between 25 and 30 seconds is particularly advantageous, since it offers a balance between a sufficiently early measurement (taking advantage of the high signal level) but sufficiently distanced from the beginning of the time to dismiss any transient effect.

In particular embodiments, the first compound is the original sample, and the second compound is an enzyme. In particular embodiments, the enzyme is a peroxidase, such as catalase, for example.

Peroxidase enzymes, such as catalase, rapidly eliminate hydrogen peroxide, allowing a reference value of zero to be obtained in the modified sample. Nevertheless, it is understood that when the compound to be measured is another different compound, any enzyme which selectively and efficiently breaks down said compound which may have been measured by an electrochemical technique falls within the scope of the invention, since use thereof fits perfectly in the defined methodology.

In particular embodiments, the first compound comprises a concentration of the compound for analysis, and the second compound is the original sample.

The concentration of the compound for analysis can be adjusted at will so as to optimize the measurement results. In this case, the first measurement will correspond with the measurement of a known concentration of the compound for analysis, whereas the second measurement will correspond with the sum of the known concentration and the concentration of the original sample.

In particular embodiments, the compound for analysis is hydrogen peroxide.

As indicated above, it is a compound present in many applications, so the measurement of its concentration may be very useful. Furthermore, the measurement obtained can also be used to quantify enzyme activity, for example, peroxidase activity. Furthermore, if selective inhibitors are used, different types of enzymes could be considered.

In particular embodiments, the electrochemical transducer is a fungible strip and comprises a working electrode, a pseudo-reference electrode, and an auxiliary electrode made of carbon modified with Co-Phthalocyanine.

The use of this electrode allows obtaining an increased signal to be obtained, which signal filters out the noise coming from a large number of compounds. The measurement is therefore particularly useful for measuring the concentration of some compounds, such as hydrogen peroxide or thiols.

Furthermore, the method of the invention enables depositing the modified sample in a fungible strip. Therefore, a portable device would be in charge of performing the steps of applying voltage to the sample and calculating the first and second concentrations.

In particular embodiments, the second transducer is the first transducer after having been cleaned.

There is the option of using two different transducers or of cleaning the first transducer to use it again in the second measurement. The preference for one option or the other will depend on the means available in each case.

In particular embodiments, after mixing the first compound with the second compound, the method comprises the additional step of leaving the modified sample to incubate for at least 5 seconds.

Quality of the measurement is thereby improved, ensuring homogeneity of the mixture.

In particular embodiments, the original sample has a volume comprised between 25 and 55 µl.

This volume is suitable for ensuring an optimal measurement.

In particular embodiments, the method includes a preliminary transformation step for transforming the original sample, in which an initial compound for analysis is chosen and said initial compound for analysis is transformed into another compound, which will be used as the compound for analysis in the remaining steps of the method.

What this stem means is that even though the method is optimized for measuring the concentration of a given compound for analysis, such as hydrogen peroxide for example, there is a possibility of measuring the concentration of other compounds.

For example, there is an original sample in which the content of glucose (the initial compound for analysis) is to be measured. To that end, this prior step of transforming all the glucose into hydrogen peroxide is performed. This hydrogen peroxide is what acts as a compound for analysis in the remaining steps of the method. The steps of the method thereby allow the concentration of the compound for analysis (i.e., hydrogen peroxide) to be calculated, which allows obtaining the concentration which the initial compound for analysis (i.e., glucose) had in the original sample before the preliminary transformation step.

In an additional inventive aspect, the invention relates to a measurement apparatus comprising means for carrying out the steps of a method according to the first inventive aspect.

### DESCRIPTION OF THE FIGURES

A brief description of each of the figures used to complete the following description of the invention is provided below. Said figures relate to the state of the art or to preferred embodiments of the invention, which are presented as non-limiting examples thereof.
Figure 1 shows the steps of a first method according to the invention.
Figure 2 shows the graphic evolution of intensity in nanoamperes with respect to time in measurements performed in a method according to the invention.
Figure 3 shows the relationship between the differential intensity and the concentration of hydrogen peroxide in a method according to the invention.
Figure 4 shows the steps of a second method according to the invention.

### DETAILED DESCRIPTION OF THE INVENTION

An example of preferred embodiment of the present invention, provided for the purpose of illustrating but not limiting the invention, is described below.

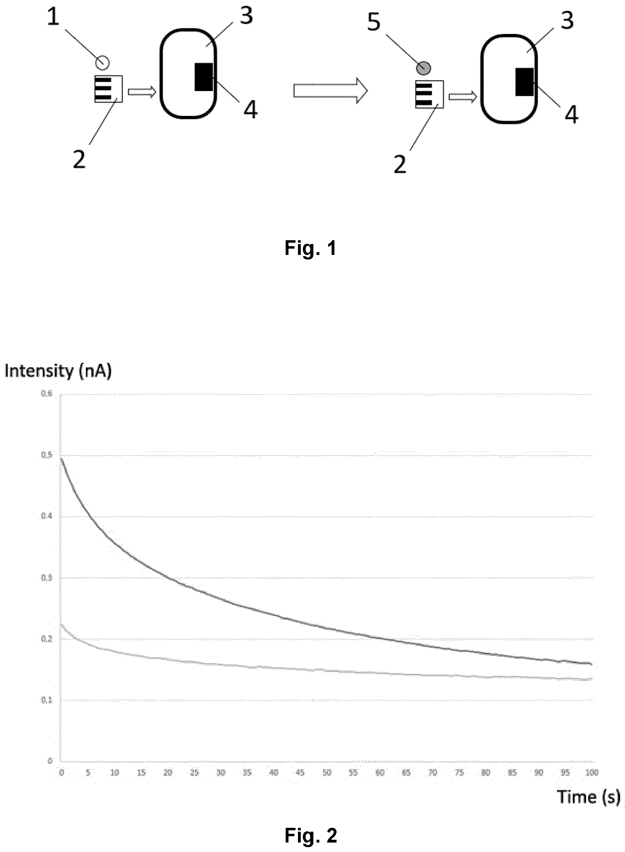

Figure 1 shows the steps of a first method according to the invention.

In this first method, an original food sample 1 in respect of which the concentration of hydrogen peroxide is to be obtained is used as the starting material.

To that end, 50 µl of the original sample 1 are placed in a fungible strip 2 having three electrodes: a working electrode, a pseudo-reference electrode, and an auxiliary electrode made of carbon modified with Co-Phthalocyanine.

Once the sample is in place, the fungible strip 2 is introduced in a portable measurement device 3 where a voltage of 0.4 V is applied to the working electrode by means of a potentiometer 4 according to an amperometry method. In this process, the mean of the current intensity obtained is obtained between 25 and 30 seconds.

Next, another 50 µl of the original sample 1 are taken and a catalase solution 7 is added thereto. The mixture 5 is left to incubate for one minute and placed on a second fungible strip 2 identical to the first strip, is introduced in the portable measurement device 3 again, and an amperometry is performed again under the same terms as above, obtaining a second value that is the mean of the current intensity obtained between 25 and 30 seconds.

The difference in nA between the mean intensity obtained in the first measurement and the mean intensity obtained in the second measurement is directly proportional to the concentration of hydrogen peroxide in the original sample, because the difference between the first and the second sample is the action of the catalase, which has eliminated the concentration of hydrogen peroxide from the sample.

Therefore, by performing a second amperometry under the same terms as the first one, the difference will be due only to the difference in the concentration of hydrogen peroxide between both samples, which is precisely what is to be measured. Taking into account that the concentration is zero in the second sample, since it has been eliminated by the catalase, the difference in concentration will provide the concentration of hydrogen peroxide in the unmodified original sample.

As will be evident to one skilled in the art, this method can be reproduced using another different enzyme for measuring the concentration of another different compound, provided that the enzyme is suitable for selectively and effectively breaking down said compound the concentration of which is to be measured.

Figure 2 shows the graphic evolution of intensity in nanoamperes with respect to time in each of the two measurements.

At the top line of the graph, represented in a dark line, the evolution of intensity with respect to time in the first measurement performed on the original sample with the concentration of hydrogen peroxide to be measured is represented.

In this evolution, several measurements were taken between 25 and 30 seconds, obtaining a mean intensity value of 0.2736 mA.

This value alone does not allow the concentration of hydrogen peroxide to be calculated, since the form of the intensity may be due to the many and very different compounds.

The bottom line of the graph, represented in a lighter colored line, shows the evolution of intensity over time in the second measurement performed on the mixture of the original sample and catalase, once it has been left to stand.

In this evolution, several measurements were taken between 25 and 30 seconds, obtaining a mean intensity value of 0.1600 mA.

The difference between both values means gives a differential intensity of 0.1136 mA.

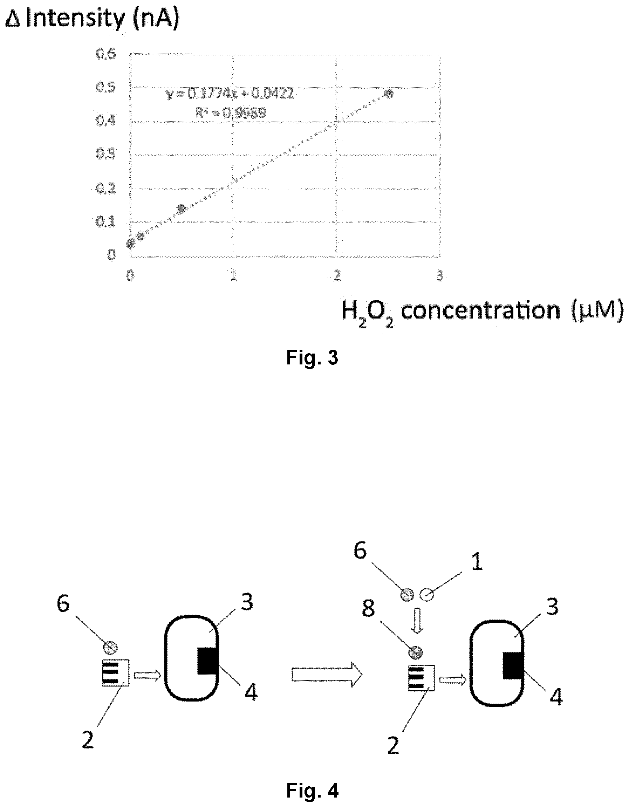

Figure 3 shows the relationship between this differential intensity and the concentration of the eliminated hydrogen peroxide. As can be observed, the relationship is virtually linear, so the differential intensity, obtained as the difference between the mean of the measurements before applying the catalase and the mean of the measurements after applying the catalase, allows the concentration of hydrogen peroxide in the original sample to be deduced in a simple manner.

There is a variant of this method. For example, if what is to be measured in a sample is the concentration of glucose, the method particularly designed for hydrogen peroxide simply with a prior step of transforming all the glucose into hydrogen peroxide can be used.

Therefore, once that prior step has been performed, it provides an altered original sample having a concentration of hydrogen peroxide that is equivalent to the concentration of glucose of the original sample. By applying the method described above to the altered original sample, the concentration of hydrogen peroxide of the altered original sample will be obtained and used to readily deduce concentration of glucose of the original sample.

Figure 4 shows the steps of a second method according to the invention.

In this case, a variant of the preceding method is used. In this case, it is also an original food sample in respect of which the concentration of hydrogen peroxide is to be measured.

However, in this case, the first step comprises placing a solution 6 with a known concentration of hydrogen peroxide in the fungible strip 2. When amperometry is performed under the same terms as in the preceding example, a first measurement of the mean intensity obtained is obtained between 25 and 30 seconds.

The following step consists of mixing the original sample 1 with the first solution 6 and performing amperometry again on the resulting mixture 8.

In this case, the second measurement obtained will refer to a mixture in which the concentration of hydrogen peroxide will be the sum between the known concentration of the first solution and the concentration of the sample the concentration of which is to be known (obviously corrected for the increase in volume of the mixture).

Therefore, when performing the second amperometry, it is easy to obtain the concentration of hydrogen peroxide of the original sample by means of a mean intensity different operation.

## Claims

1. A method for measuring a concentration of a compound for analysis in an original sample, the method comprising the steps of
placing a first compound (1, 6) in a first electrochemical transducer (2) containing a working electrode, a reference or pseudo-reference electrode, and an auxiliary electrode;
measuring a first concentration by means of applying a potential in the electrochemical transducer (2);
mixing the first compound (1, 6) with a second compound (1, 7), where the first or the second compound is a part of the original sample (1), obtaining a modified sample (5, 8), where the modified sample (5, 8) has a concentration of the compound for analysis different from that of the original sample due to the action between the first compound and the second compound;
placing the modified sample in a second electrochemical transducer (2) containing a working electrode, a reference or pseudo-reference electrode, and an auxiliary electrode;
measuring a second concentration by means of applying a potential in the electrochemical transducer (2);
performing an operation between the first and the second concentration to obtain the concentration of the compound for analysis in the original sample.

2. The method according to claim 1, wherein the step of measuring the first or the second concentration comprises
applying a voltage to the working electrode by means of a potentiostat (4) according to an amperometry method, said voltage being comprised within the range between 0.35 and 0.45 V;
obtaining a mean of several current intensity measurements obtained in a given time range.

3. The method according to claim 2, wherein the time range is comprised in the range between 20 and 40 seconds, specifically between 25 and 30 seconds.

4. The method according to any of the preceding claims, wherein the first compound is the original sample, and the second compound is an enzyme.

5. The method according to claim 4, wherein the enzyme is catalase (7).

6. The method according to any of claims 1 to 3, wherein the first compound comprises a concentration of the compound (6) for analysis and the second compound is the original sample (1).

7. The method according to any of the preceding claims, wherein the compound for analysis is hydrogen peroxide.

8. The method according to any of the preceding claims, wherein the electrochemical transducer (2) is a fungible strip and comprises a working electrode, a pseudo-reference electrode, and an auxiliary electrode made of carbon modified with Co-Phthalocyanine.

9. The method according to any of the preceding claims, wherein the second transducer is the first transducer after having been cleaned.

10. The method according to any of the preceding claims, wherein, after mixing the first compound with the second compound, the method comprises the additional step of leaving the modified sample (5, 8) to incubate for at least 5 seconds.

11. The method according to any of the preceding claims, wherein the original sample (1) has a volume comprised between 25 and 55 pl.

12. The method according to any of the preceding claims, including a preliminary transformation step for transforming the original mixture, wherein an initial compound for analysis is chosen, and said initial compound for analysis is transformed into another compound, which will be used as the compound for analysis in the remaining steps of the method.

13. A measurement apparatus (3) comprising means for carrying out the steps of a method according to the preceding claims.
